# EUROPEAN PATENT SPECIFICATION

(11) **EP 1 512 622 B1**
(45) Date of publication and mention of the grant of the patent: **10.08.2011**
(21) Application number: 04425638.6
(22) Date of filing: 27.08.2004
(51) Int. Cl.: B63C 11/12, A63B 33/00, A44B 11/20

(54) **Device for regulating the length of a scuba diving mask strap**
Vorrichtung zum Einstellen der Länge des Kopfbandes einer Tauchermaske
Dispositif pour régler la longeur de la bande d'un masque de plongée

(30) Priority: 03.09.2003 IT FI20030082 U
(43) Date of publication of application: 09.03.2005
(73) Proprietor: Cressi-Sub S.p.A., 16165 Genova (IT)
(72) Inventor: Godoy, Carlos, 16148 Genova (IT)
(74) Representative: Bardini, Marco Luigi

(56) References cited:
- DE-A1- 4 329 492
- US-A- 4 727 630
- US-A- 5 267 679
- US-A- 5 697 107
- US-A- 5 956 778
- US-B1- 6 219 889

## Description

The present invention relates generally to a device for regulating the length of the strap normally attached to a scuba diving mask to enable it to be fitted over the diver's head.

Scuba diving masks are known to comprise a frame, to which one or two lenses are attached with a watertight seal, a soft facepiece extending from the edge of the frame to create a seal against the diver's face, and a flexible strap, the two ends of which are connected to the two opposite sides of the frame, and the length of the strap is adjustable so as to exert sufficient tension to ensure its stable positioning against the diver's face and an adequate watertightness of the facepiece.

The strap's length is still commonly regulated by means of a buckle, although this is somewhat impractical to use, especially when the diver is wearing gloves, or has cold or wet hands. It is also common to find fastenings that enable the length of the strap to be adjusted without the diver needing to take off the mask. A known solution of this kind involves a strap whose ends wrap around pivots located on fork-shaped side portion of the mask. The strap has transverse stop ribbing, which is engaged by a stop tooth projecting from a lever pressed elastically against said ribs. By overcoming its elastic resistance, this lever can be raised by the user, without taking off the mask, to enable the strap to slide into a new position. Said elastic resistance is created by a spring in some cases, while in others the resilience of the lever itself is exploited, as it is flexed to detach the tooth from the strap to enable the latter to slide. Even with solutions of this type, however, although it is not necessary to take off the mask, regulating the strap is still uncomfortable and tricky, especially if it is done with cold, wet hands - and it cannot be done while wearing gloves because a finger tip is needed to lift the lever to which the stop tooth engaging the strap's ribbing is attached.

The US patent no. 5956778, in the name of the same Applicant, which document is considered to represent the closest prior art, discloses the following features of the independent claim 1: a mask comprising a strap and a device for regulating the length of the strap of the mask, said mask further comprising a frame for supporting one or two lenses and two fork-shaped portions extending from either side of said frame, each containing a pivot around which an end portion of the strap is wrapped, said end portion of the strap being formed with a plurality of transverse stop ribs on one side, and said regulating device comprising a stop lever housed inside each of said fork-shaped portions and a tooth extending from said lever for engaging with one of said ribs, said lever comprising a substantially U-shaped member with a base and a tongue extending from said base so that it is coplanar and cantilevered inside it, said tongue having a free end and said tooth extending transversely from said tongue.

US patent no. 5956778 discloses a particularly compact device for regulating the strap, suitable for use on swimming goggles. The device comprises a stop lever and a tooth extending from the lever that engages on a rib on the strap wrapped around an underlying pivot integrally attached to a side portion on the goggles frame. The stop lever comprises a first arm (in particular, in the form of a fork), having a first end pivotally connected to the side portion of the frame and a second end abutting against the bottom of a seat provided in the side portion of the frame, and a second arm, from which said tooth extends transversely, having a free first end for its operation and a second end rigidly connected to the second end of the first arm. In particular, the second arm is a tongue that extends so as to be cantilevered and coplanar to the fork forming the first arm, and it has a raised portion at its free end that can be used to lift the tongue and thus disengage the transverse tooth from the rib on the strap, thereby enabling it to slide.

This solution also suffers from the same drawback as other known solutions, however, i.e. it is difficult to operate the stop lever.

The object of the present invention is to provide a scuba diving mask with a device for regulating the length of the strap of the mask that can be operated more easily and comfortably than the known devices, even wearing gloves.

This object is achieved by the scuba diving mask with a device for regulating the length of the strap of the mask with the essential characteristics are set forth in claim 1. Further important features are set forth in the dependent claims.

The invention will now be illustrated in greater detail in the following description of an embodiment thereof, which is to be intended only as a non-limiting example with reference to the enclosed drawings, wherein:
figure 1 shows a schematic side view of a scuba diving mask using the device for regulating the length of the strap according to the invention;
figure 2 is a perspective view of the regulating device according to the invention;
figure 3 is a plan view of the regulating device according to the invention;
figure 4 is a view from above of the side portion of a scuba diving mask frame housing the device according to the invention;
figure 5 is a side view of the detail shown in figure 4, seen from the direction indicated by the arrow F;
figure 6 is a rear view of the detail shown in figure 4, seen from the direction indicated by the arrow G.

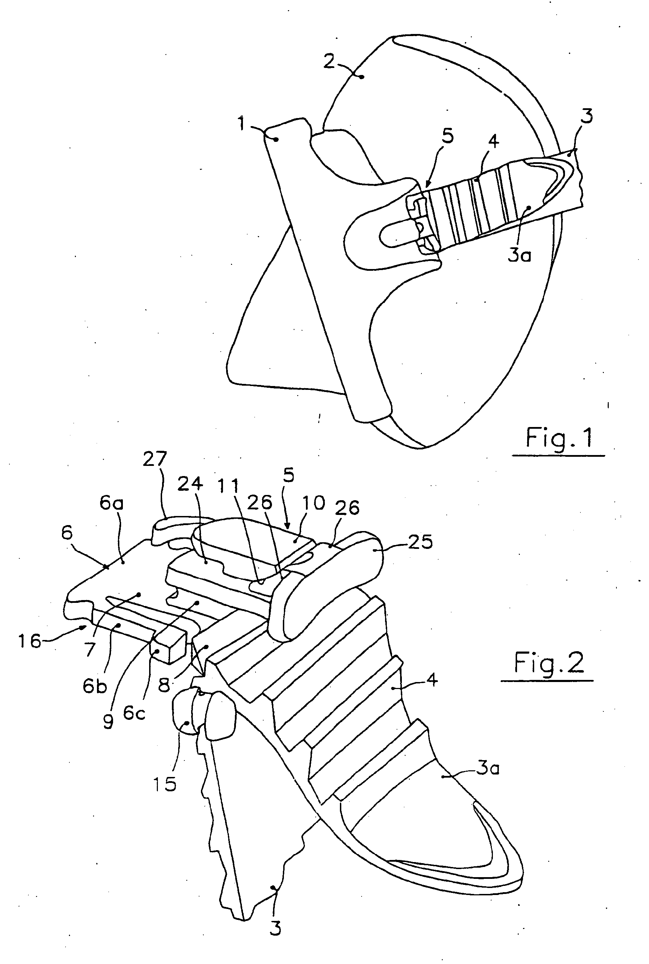

With reference to figure 1, the numeral 1 is used to indicate a scuba diving mask frame providing a watertight support for one or two lenses (not shown) and a facepiece 2 made of a soft material extending from the edge of the frame and suitable for adapting the mask to the diver's face, while also ensuring a watertight seal against the face, achieved by adjusting the tightness of the mask against the face by means of a belt or strap 3 that wraps around the diver's head.

The strap 3 has a set of transverse ribs 4 on one side of its terminal portions 3a, which engage in a device for fastening and regulating the length of said strap generically indicated by the numeral 5.

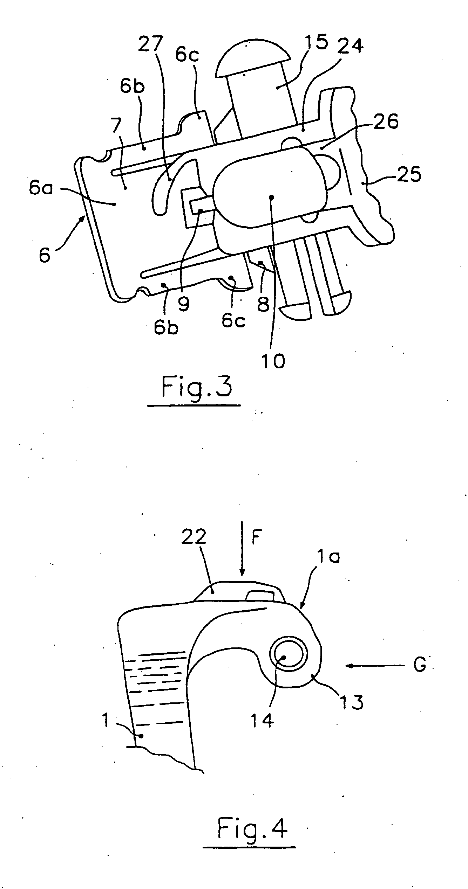

As illustrated in detail in figures 2 and 3, the regulating device 5 comprises a first, substantially U-shaped member 6, composed of a base 6a and a pair of brackets 6b whose expanded tips 6c extend from the base, remaining coplanar thereto, and a tongue 7 extending from the base 6a of the U-shaped member 6 between the two brackets 6b in a position substantially coplanar to said brackets. At the free end of the tongue 7 there extends a transverse tooth 8. The assembly comprising the U-shaped member 6, the tongue 7 and the corresponding tooth 8 constitutes the stop lever 16 for the regulating device according to the present invention.

On the side opposite the transverse tooth 8, there is a column 9 extending from the tongue 7, that is enlarged at one end to form a head 10, on which head (on the side opposite the base 6a of the U-shaped member 6) there is a surface 11 sloping towards the tongue 7.

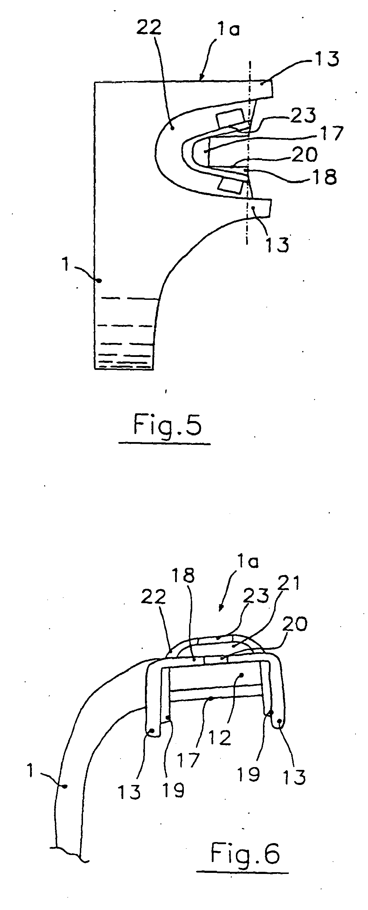

With reference also to figures 4, 5 and 6, the stop lever 16 is mounted inside a seat 12 in a fork-shaped side portion 1a that projects from the frame 1. This portion 1a comprises a pair of fins 13, with a corresponding pair of coaxial holes 14, with a pivot 15 which is snap-fixed into said holes and the terminal portion 3a of the strap 3 is wrapped around said pivot.

The seat 12 comes between the two fins 13 and two substantially parallel walls 17 and 18 extending between them. The depth of the seat 12 is such that the base 6a of the U-shaped member 6 abuts against the bottom of said seat and the expanded tips 6c on its brackets 6b abut against ribbing 19 provided along the fins 13 at the inlet to the seat 12, so that once the U-shaped member 6 has snapped into the seat 12, it remains locked therein.

The wall 18, forming the outer side of the seat 12 has a longitudinal slot 20, which is occupied by the column 9 extending from the tongue 7. A further seat 21 is provided beyond the wall 18, contained on the outside by a wall 22 with a relevant longitudinal slot 23 lying above the slot 20 and containing the column 9 so that its enlarged end, or head, 10 projects therefrom.

A plate 24, with one end 25 enlarged in the shape of a button, projects from and is slidingly engaged around the column 9 within the seat 21. Between the button-shaped end 25 and the head 10 situated on the end of the column 9, there is a sloping wall 26 corresponding to the wall 11 under the head 10 and sloping at the same angle. Pressing the button-shaped end 25 of the plate 24 causes the latter to slide in the seat 21, thereby bringing the sloping walls 26 and 11 into contact. Since the head 10 is integral to the lever 16, which cannot slide within its seat 12, said head is forced to slide along the sloping wall 26 of the button 25 with its sloping wall 11, and is thereby lifted. As the head 10 rises, it lifts the tongue 7 and consequently disengages the tooth 8 from the rib 4 on the strap 3, thus enabling the strap to slide around the pivot 15. To return the tooth 8 automatically to engage with the rib 4 when the pressure on the button 25 is released, the plate 24 is elastically forced against the bottom of the seat 21. For this purpose, an arm 27 extends from the end of the plate 24 opposite the button 25 and said arm is bent over to form an arc and suitable for abutting against the bottom of the seat 21 as it flexes under the effect of the pressure induced by the sliding of the plate 24, then returning to its undeformed condition when the pressure is released and thus returning the button 25 to its initial position, when the head 10 is consequently lowered and the tooth 8 re-engages with a rib 4. To prevent the plate 24 from sliding out of its seat 21 at the end of its return stroke, it abuts against the column 9 around which it is installed.

It should be noted that the height of the column 9 is designed so that a minimal effort is needed to make the plate 24 slide and the head 10 rise.

From the above, it is clear that the device for regulating the length of the strap on a scuba diving mask fully satisfies the established aims. In fact, the device can easily be adjusted without taking off the mask, even if the diver is wearing gloves, since the lever that disengages the tooth from the ribbing on the strap is lifted by taking action not directly on the lever, using a tugging action parallel to the movement that disengages the tooth (as in the known solution), but on a button connected to the lever and consequently much more readily accessible in that pressure is exerted in a direction perpendicular to said tooth movement, i.e. substantially parallel to the plane on which the end of the strap lies.

It is also worth pointing out that, although the regulating device according to the present invention has been described in relation to a scuba diving mask, it can clearly also be equally advantageously applied to a pair of swimming goggles.

## Claims

1. A scuba diving mask comprising a strap (3) and a device (5) for regulating the length of the strap (3) of the mask, said mask further comprising a frame (1) for supporting one or two lenses and two fork-shaped portions (1a) extending from either side of said frame, each containing a pivot (15) around which an end portion (3a) of the strap (3) is wrapped, said end portion (3a) of the strap being formed with a plurality of transverse stop
ribs (4) on one side, and said regulating device (5) comprising a stop lever (16) housed inside each of said fork-shaped portions (1a) and a tooth (8) extending from said lever for engaging with one of said ribs (4) said lever (16) comprising a substantially U-shaped member (6) with a base (6a) and a tongue (7) extending from said base (6a) so that it is coplanar and cantilevered inside it, said tongue (7) having a free end and said tooth (8) extending transversely from the free end of said tongue (7); said device comprising further, on the side opposite said tooth (8), a head (10), which extends from said tongue (7) and has a sloping surface (11), against which a wedge-shaped portion (26) of a button (25) abuts, said button (25) sliding on a plane parallel to said U-shaped member (6) with respect to said fork-shaped portion (1a) and the direction of extension of said tongue (7), whereby, when said button (25) is pressed, said head (10) is caused to slide away from said U-shaped member (6), with a consequent lifting of said tongue (7) and the disengagement of said tooth (8) from said stop rib(4).

2. The mask according to claim 1, wherein each of said fork-shaped portions (1a) contains a first seat (12) for snap-fixing said substantially U-shaped member (6) and a second seat (21) over said first seat for slidingly housing a plate (24) extending from said button (25), said head being formed at the end of a column (9) extending from said tongue (7) and passing through said second seat (21), so that said head projects above the seat (21).

3. The mask according to claims 1 or 2, wherein said button is elastically forced against the bottom of said second seat (21).

4. The mask according to claim 3, wherein a curved arm (27) extends from the sliding plate (24) of said button and abuts flexibly up against the bottom of said second seat (21).

5. The mask according to any of the previous claims, in which said plate (24) is arranged around said column (9), it slides perpendicularly to the column (9) and it abuts against said column (9), thus being prevented from sliding out of said second seat (21).

6. The mask according to any of the previous claims, in which said fork-shaped portion (1a) is composed of two parallel fins (13) with two coaxial holes (14) for supporting said pivot (15), said first (12) and second seat (21) extending one above the other, between said fins and delimited by three substantially parallel walls (17, 18, 22), two of which each have a slot (20, 23) for housing said column (9) on said tongue (7).

## Patentansprüche

1. Tauchermaske, enthaltend ein Band (3) und eine Vorrichtung (5) zum Regulieren der Länge des Bandes (3) der Maske, wobei die Maske ferner einen Rahmen (1) zum Halten von einer oder zwei Linse(n) und zwei gabelförmige, sich von beiden Seiten des Rahmens aus erstreckende Teile (1a) enthält, von denen jedes einen Achszapfen (15) enthält, um welchen ein Endteil (3a) des Bandes (3) gewickelt ist, wobei der Endteil (3a) des Bandes auf einer Seite mit einer Mehrzahl von querverlaufenden Halterippen (4) ausgebildet ist und die Reguliervorrichtung (5) einen Haltearm (16), der innerhalb jedes der gabelförmigen Teile (1a) untergebracht ist, und einen Zahn (8) enthält, der sich von dem Arm aus erstreckt, um mit einer der Rippen (4) in Eingriff zu sein, wobei der Arm (16) ein im Wesentlichen U-förmiges Element (6) mit einer Basis (6a) und eine Zunge (7) enthält, die sich von der Basis (6a) aus erstreckt, so dass sie darin koplanar und freitragend ist, wobei die Zunge (7) ein freies Ende hat und sich der Zahn (8) quer von dem freien Ende der Zunge (7) aus erstreckt, wobei die Vorrichtung ferner auf der Seite entgegengesetzt dem Zahn (8) einen Kopf (10) enthält, der sich von der Zunge (7) aus erstreckt und eine geneigte Oberfläche (11) hat, gegen welche ein keilförmiger Teil (26) eines Knopfes (25) anliegt, wobei sich der Knopf (25) an einer Ebene parallel zu dem U-förmigen Element (6) bezüglich dem gabelförmigen Teil (1a) und in der Ausdehnungsrichtung der Zunge (7) verschiebt, wodurch, wenn der Knopf (25) gedrückt wird, der Kopf (10) veranlasst wird, sich von dem U-förmigen Element (6) weg zu verschieben, mit einer konsequenten Anhebung der Zunge (7) und dem Ausrücken des Zahnes (8) aus der Halterippe (4).

2. Maske nach Anspruch 1, wobei jedes der gabelförmigen Teile (1a) einen ersten Sitz (12) zum Schnappbefestigen des im Wesentlichen U-förmigen Elementes (6) und einen zweiten Sitz (21) über dem ersten Sitz, um eine Platte (24) verschiebbar unterzubringen, die sich von dem Knopf (25) aus erstreckt, wobei der Kopf an dem Ende einer Stütze (9) ausgebildet ist, die sich von der Zunge (7) aus erstreckt und durch den zweiten Sitz (21) hindurch geht, so dass der Kopf über den Sitz (21) vorsteht.

3. Maske nach Anspruch 1 oder 2, wobei der Knopf elastisch gegen den Boden des zweiten Sitzes (21) gedrängt wird.

4. Maske nach Anspruch 3, wobei sich ein gekrümmter Arm (27) von der Verschiebeplatte (24) des Knopfes aus erstreckt und flexibel gegen den Boden des zweiten Sitzes (21) anliegt.

5. Maske nach einem der vorhergehenden Ansprüche, bei der die Platte (24) um die Stütze (9) herum angeordnet ist, sie sich senkrecht zu der Stütze (9) verschiebt und sie gegen die Stütze (9) anliegt, wodurch sie an einem Verschieben aus dem zweiten Sitz (21) gehindert ist.

6. Maske nach einem der vorhergehenden Ansprüche, bei der das gabelförmige Teil (1a) aus zwei parallelen Finnen (13) mit zwei koaxialen Löchern (14) zum Halten des Achszapfens (15) besteht, wobei der erste (12) und zweite Sitz (21) sich einer über dem anderen zwischen den Finnen und durch drei im Wesentlichen parallele Wände (17, 18, 22) begrenzt erstrecken, von denen zwei je einen Schlitz (20, 23) zum Unterbringen der Stütze (9) an der Zunge (7) haben.

## Revendications

1. Un masque de plongée pour scaphandre autonome comprenant une bande (3) et un dispositif (5) pour régler la longueur de la bande (3) du masque, ledit masque comprenant en outre une monture (1) pour soutenir un ou deux verres et deux parties en forme de fourche (1a) s'étendant de chaque côté du dit cadre, chacune contenant un pivot (15) autour duquel est enroulée une partie d'extrémité (3a) de la bande (3), ladite partie d'extrémité (3a) de la bande comportant, sur une face, une pluralité de nervures d'arrêt transversales (4), et ledit dispositif de réglage (5) comportant un levier d'arrêt (16) logé à l'intérieur de chacune des dites parties en forme de fourche (1a) et une dent (8) s'étendant à partir du dit levier pour coopérer avec une des dites nervures (4), ledit levier (16) comportant un élément sensiblement un forme de U avec une base (6a) et une languette (7) s'étendant à partir de ladite base (6a) de façon qu'elle soit coplanaire et en porte-à-faux à l'intérieur de celui-ci, ladite languette (7) ayant une extrémité libre et ladite dent (8) s'étendant transversalement à partir de l'extrémité libre de ladite languette (7) ; ledit dispositif comprenant en outre, sur le côté opposé à ladite dent (8), une tête (10) qui s'étend à partir de ladite languette (7) et qui a une surface en plan incliné (11), contre laquelle vient buter une partie en coin (26) d'un bouton (25), ledit bouton (25) glissant sur un plan parallèle au dit élément en forme de U (6) par rapport à ladite partie en forme de fourche (1a) et dans la direction d'extension de ladite languette (7), de sorte que, quand ledit bouton (25) est pressé, ladite tête (10) s'éloigne de l'élément en forme de U (6) en glissant, ce qui a pour conséquence le relevage de ladite languette (7) et le dégagement de ladite dent (8) de ladite nervure d'arrêt (4).

2. Le masque selon la revendication 1, où chacune des dites parties en forme de fourche (1a) contient un premier siège (12) pour fixer par enclipsage ledit élément sensiblement en forme de U (16) et un second siège (21) au-dessus du dit premier siège pour loger de façon coulissante une patte (24) s'étendant à partir du dit bouton (25), ladite tête étant formée à l'extrémité d'une colonne (9) s'étendant à partir de ladite languette (7) et passant à travers ledit second siège (21), de sorte que ladite tête se projette au-dessus du siège (21).

3. Le masque selon l'une des revendications 1 et 2, où ledit bouton est élastiquement engagé en force contre le fond du dit second siège (21).

4. Le masque selon la revendication 3, où un bras incurvé (27) s'étend à partir de la patte coulissante (24) du dit bouton et vient buter en flexion vers le haut contre le fond du dit second siège (21).

5. Le masque selon l'une quelconque des revendications précédentes, dans lequel ladite patte (24) est agencée pour passer autour de ladite colonne (9), glisse perpendiculairement à la colonne (9) et vient buter contre ladite colonne (9) étant ainsi empêchée de glisser hors du dit second siège (21).

6. Le masque selon l'une quelconque des revendications précédentes, dans lequel ladite partie en forme de fourche (1a) se compose de deux ailettes parallèles (13) avec deux trous coaxiaux (14) pour soutenir ledit pivot (15), lesdits premier (12) et second (21) sièges s'étendant l'un au-dessus de l'autre entre lesdites ailettes et étant délimités par trois parois sensiblement parallèles (17, 18, 22), deux de celles-ci ayant chacune une fente (20, 23) pour loger ladite colonne (9) sur ladite languette (7).
